Europäisches Patentamt

(19) European Patent Office

Office européen des brevets

(11) Publication number: **0 067 922**

**A1**

(12) # EUROPEAN PATENT APPLICATION

(21) Application number: **81830100.4**

(22) Date of filing: **22.06.81**

(51) Int. Cl.³: **A 61 N 1/14**

(43) Date of publication of application:
**29.12.82 Bulletin 82/52**

(84) Designated Contracting States:
**AT BE CH DE FR GB IT LI LU NL SE**

(71) Applicant: **Tonlorenzi, Giorgio**
**Via Croce, 70**
**I-54038 Marina di Montignoso Massa Carrara(IT)**

(72) Inventor: **Tonlorenzi, Giorgio**
**Via Croce, 70**
**I-54038 Marina di Montignoso Massa Carrara(IT)**

(74) Representative: **Sassatelli, Franco**
**c/o INIP Ufficio Internazionale Brevetti per Deposito di**
**Brevetti e Marchi via Mazzini, 170**
**I-40139 Bologna(IT)**

(54) Device for balancing again the energetic equilibrium of human body.

(57) For carrying out the re-balancing of one or more organs of the human body, a discharging device is used which, by means of bandaging connections on the head and ankle or other part, discharges the accumulation kinetic energy to the floor by a point driven into it.

In particular, if fitted on the head by means a circular bandage, the device permits to obviate hemicranias and neuralgies in general and to carry out the discharge of the nervous tension, soothing in this way weariness and re-generating the nervous balance.

FIG.2

EP 0 067 922 A1

- 1 -

"Device for balancing again the energetic equilibrium of human body organs"

The invention refers to a device which substantially allows to carry out the energetic re-balancing of one or more organs of the human body, and in this way to obviate hemicranias, neuralgies and different uneasiness subsequent to the surcharge of the physical and psychical motion energy and particularly to operate as regenerator of the nervous system.

It is well known that the pain felt at a human organ may be caused either by a pathological condition, or by a reflected effect, or by accumulation kinetic energy which is altering its biological equilibrium. The invention is referring to this last casualness. Through the human body, the electromagnetic energy (bioenergy) is passing as an effect of the earth magnetism and gets out from the tips, hand and feet fingers. Owing to the garment worn, however, which have the task to keep back the energy in order to maintain the body temperature within tolerable values, this is prevented.

Moreover, the ground of the present living surrounding is in general a bad energy absober. It is well known, that any person is able to re-balance the bioenergetic equilibrium of another human body. By putting his hands in open conversion on the suffering organ, the energetic flux is transmitted which the percipient feels in the form of warm vibrations. However, this requires the presence of a he althy person at disposal and a prolonged engagement of the opera tor.

Beside that, the contact is necessary of the operator's hands on the percipient's organ in either epidermic way or at a distance, which condition turns out unpleasant. In the pranotherapy, a bio energetic flux is generated which passes trough the human body balancing again the bioenergetic conditions of the treated organ, while the energy excess is discharged trough the jointed feet on the ground, with a flux continuity into the same.

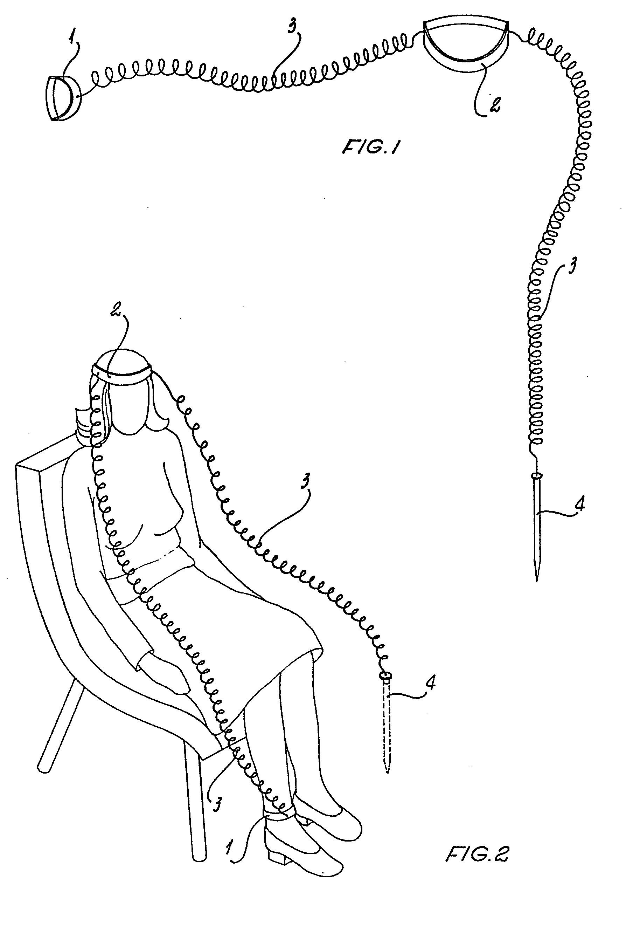

The invention permits an autogenous therapy re-balancing the bioenergetic condition that the percipient can carry out in rest con dition using a ground with good conductivity. A form of execution is illustrated in a quite indicative way and, as such, not limita ting of the procedure, in the drawings of Table 1, where Fig. 1 is the view of an apparatus showing the discharging point of the energy to be driven into the ground and that employs a discharge spiral conductor, with intermediate bandaging device on the head and end bandaging one at the ankle. Fig. 2 is the view of an ope-rator percipient in sitting position with applied device and point driven into the ground. The version foresees bandaging apparatus 1 that can be fitted on the ankle and another bandaging device 2 to be fitted on the head, both to be carried out with material having good conductivity and with retaining part to permit the epidermic adherence, connected with spiral conductors 3 which rea ches point 4 in copper, gold or other material having a high bioenergetic conductivity. In particular, the form of the bandaging as well of the retaining devices aiming at permitting epidermic adherence on the binding part, can be carried out in different wa ys.

- 3 -

The device can be executed in different forms and with different materials and anything else in order.

- 1 -

Claims:

1) Device for balancing again the energetic equilibrium of human body organs, characterized by the fact that it allows an autogeno us therpy balancing again the bioenergetic condition of one or more organs of human body, in particular of the head, which percipient can autonomously carry out in sitting position using a ground with good conductivity. To do so, the operator-percipient will previously drive the point into the good conductivity ground then he will fit the conductivity pressors to his head and ankle, and putting himself in resting position, he will relax himself re leasing his mind from cares. In this condition he will keep himself during some time in relation.

2) Device for balancing again the energetic equilibrium of human body organs, according to the previous claim, characterized by the fact that the version foresees bandaging device 1 that can be fitted at the ankle and another bandaging device 2 to fit at the head, both to be carried out with epidermic contact bandages havi ng good bioenergetic conductivity and equipped with retention de vice to allow the bandaging condition with permanent contact effe ct. The said bandaging parts are connected with spiral conductor 3 which continues as far as point 4 in copper, gold or other material.

3) Device for balncing again the energetic equilibrium of human body organs, according to the above claims, characterized by the fact that, in order to allow the employ of the human mind for the orientation of the bioenergetic flux in an autogenous way, the bandaging connection id foreseen on the part on which the operator-percipient wishes to operate with the head, proceeding then to the remaining connections according to the base therapy.

4) Device for balancing again the energetic equilibrium of human body organs, form of execution as here described and illustrated in the drawings of Table 1.

FIG.1

FIG.2

European Patent Office

**EUROPEAN SEARCH REPORT**

Application number

EP  81 83 0100

## DOCUMENTS CONSIDERED TO BE RELEVANT

| Category | Citation of document with indication, where appropriate, of relevant passages | Relevant to claim | CLASSIFICATION OF THE APPLICATION (Int. Cl. ³) |
|---|---|---|---|
| X | US-A- 620 679 (SHRYOCK) *Figure; claim 1* | 1-4 | A 61 N 1/14 |
| X | US-A-3 596 134 (BURKE) *Column 2, lines 19-39; figure 1* | 1-4 | |
| A | DE-U-7 515 904 (TILLEN) *Abstract* | 2 | |
| A | GB-A-2 025 237 (OLIVER) *Page 2, lines 32-35; claims 23 and 24* | 2 | |
| E | DE-A-3 013 119 (PROTLERMO) *Page 3, two last paragraphs; page 4* | 1-4 | |

TECHNICAL FIELDS SEARCHED (Int. Cl. ³)

A 61 N

The present search report has been drawn up for all claims

| Place of search | Date of completion of the search | Examiner |
|---|---|---|
| THE HAGUE | 06-05-1982 | SIMON J.J.E. |